# EUROPÄISCHE PATENTANMELDUNG

(11) **EP 0 855 382 A2**
(43) Veröffentlichungstag der Anmeldung: **29.07.1998**
(21) Anmeldenummer: 98100116.7
(22) Anmeldetag: 07.01.1998
(51) Int. Cl.: C07C 69/63, C07C 69/65, C07C 43/12, C07C 229/12, C07C 271/14, C07F 9/24, C07C 69/76, C07C 235/84, D06M 13/00, D06M 13/236, D06M 13/298, D06M 13/256, D06M 13/165

(54) **Fluoralkylmodifizierte Kohlenwasserstoffderivate**

(30) Priorität: 22.01.1997 DE 19702041
(71) Anmelder: Clariant GmbH, 65929 Frankfurt am Main (DE)
(72) Erfinder: Wehowsky, Frank, Dr., 84508 Burgkirchen (DE); Knaup, Wolfgang, Dr., 84508 Burgkirchen (DE); Gilsing, Hans-Detlev, Dr., 47509 Rheurdt (DE); Wagner, Roland, Dr., 12437 Berlin (DE); Prescher, Dietrich, Dr., 12435 Berlin (DE)

(57) **Zusammenfassung**

Die Erfindung betrifft langkettig fluoralkylmodifizierte Kohlenwasserstoffderivate der allgemeinen Formel

R_{F}-(CH₂)ₙ-A-D

in der
- n: 1 bis 3,
- R_{F}: geradkettige Perfluoralkylreste mit 6 bis 20 C-Atomen, vorzugsweise mit 14 bis 20 C-Atomen,
- A: eine der folgenden Gruppen: -O-C(=O)-, -O-, -O-P(=O)(Cl)-, -O-(D-)P(=O)-, -O-(O=)S(=O)- oder eine Einfachbindung und
- D: D₁: geradkettige, verzweigte oder cyclische, gesättigte oder ungesättigte, ggf. durch Stickstoff, Sauerstoff, Schwefel oder Halogen substituierte Kohlenwasserstoffe mit 2 bis 100 C-Atomen oder
D₂: Molekülgerüste des Typs Y(R¹)(R²)(R³) mit Y = gesättigte oder ungesättigte, ggf. verbrückte oder über ein Heteroatom oder eine C₁-Einheit - die durch Stickstoff, Sauerstoff, Halogen oder halogenierte Alkylreste substituiert sein kann - verknüpfte oder mehrgliedrig annelierte cyclische Kohlenwasserstoffe mit 4 bis 40 C-Atomen und R¹, R², R³ = sauerstoff-, schwefel- oder stickstoffhaltige, hydroxylierte oder nichthydroxylierte Kohlenwasserstoffreste mit 2 bis 50 C-Atomen oder Metallionen enthaltende, kovalente oder ionogene Bindungen aufweisende Substituenten in 1.2- oder 1.3-Stellung am Ring oder über zwei weitere, teilweise funktionalisierte Kohlenstoffe mit dem Ring verknüpft oder
D₃: geradkettige, verzweigte oder cyclische, gesättigte oder ungesättigte mono- oder polyhydroxylierte und/oder durch Alkylenoxideinheiten modifizierte, ggf. stickstoffhaltige Kohlenwasserstoffreste mit 2 bis 100 C-Atomen
bedeuten, sowie Verfahren zu deren Herstellung.

## Beschreibung

Die Erfindung betrifft langkettig fluoralkylmodifizierte Kohlenwasserstoffderivate und deren Herstellungsverfahren.

Für Fluoralkohole des Typs CF₃(CF₂)m(CH₂)ₙOH mit m = 3, 5, 7 und n = 2 sind Derivatisierungen mit Halogenen, halogenierten Ethern sowie halogenierten und nichthalogenierten Säurehalogeniden aus A. Sismondi, P. Abenin, L. Joncheray, A. Cambon, J. Fluorine Chem. 59 (1992) 127-32; T. Oishi, H. Morikawa, M. Fujimoto, Polym. J. 25 (1993) 41 - 8; M. Koden, K. Nakagawa, Y. Ishii, F. Funada, M. Matsuura und K. Awane, Mol. Cryst. Liq. Cryst., Lett. Sect. 6 (1989) 185 - 90; K. Waldmann DE-2 128 956; Chem. Abstr. 78 (1973) 71482e; H. Bathelt, DE-2 558 711; Chem. Abstr. 87 (1977) 117598p; F. Wehowsky, DE-3 611 302; Chem. Abstr. 108 (1988) 188957t - in den letzten drei Arbeiten auch für m = 9, 11 - bekannt.

Ferner sind für kürzerkettige Fluoralkohole mit m = 5, 7 und n = 2 auch Abwandlungen mit Phosphoroxyhalogeniden in M.-P. Krafft, P. Vierling, J. G. Riess, Eur. J. Med. Chem. 26 (1991) 545-50; A. Milius, J. Greiner, J. G. Riess, Carbohydr. Res. 129 (1992) 323 - 36; S. Gaentzler, P. Vierling, J. G. Riess, Phosphorus, Sulfur and Silicon 77 (1993) 327; J. Greiner, F. Guillod, A. Milius, J. G. Riess, Phosphorus, Sulfur and Silicon 77 (1993) 328; M.-P. Krafft, F. Giulieri und J. G. Riess, Angew. Chem. 105 (1993) 783 - 5 beschrieben.

Weiterhin sind Fluoralkohole mit m = 5, 7, 9 und n = 2 zur Silanisierung nach T. Aok, Y. Toyoshima, Polymer 33 (1992) 662 - 4; T. Aoki, Y. Toyoshima, E. Oikawa, Polym. J. 26 (1994) 1142-53, und Transesterifizierung mit aromatischen Molekülgerüsten nach U. Dahn, C. Erdelen, H. Ringsdorf, R. Festag, J. H. Wendorff P. A. Heiney, N. C. Maliszewskyj, Liq. Cryst. 19 (1995) 759 - 64 sowie zur Acylierung mit Monoanhydriden nach R. Elbert, T. Folda, H. Ringsdorf, J. Am. Chem. Soc. 106 (1984) 7687 - 92, Carbonsäuren nach L. M. Wilson, Macromolecules 28 (1995) 325-30 und Aminosäuren nach E. M. Landau, S. G. Wolf, M. Levanon, L. Leiserowitz, M. Lahav und J. Sagiv, J. Am. Chem. Soc. 111 (1989) 1436-45; Y. Ishikawa, H. Kuwahara, T. Kunitake, Chem. Lett. 1989, 1737-40; J. Am. Chem. Soc. 116 (1994) 5579-91; E. Dessipri, K. H. Yeap, D. A. Tirrell, Polym. Prepr. 36 (1995) 536 - 7 eingesetzt worden.

Alle Veröffentlichungen beschreiben nur Umwandlungen kurzkettiger Fluoralkohole, in denen m maximal gleich 11 ist. Die entsprechenden Arbeitsvorschriften beziehen sich sämtlich auf Reaktionen in Lösung, die meistens unvollständig verlaufen und nahezu immer aufwendige Reinigungs- und Trennverfahren erforderlich machen. Die Verfahren sind für die Umsetzung sehr langkettiger Fluoralkohole mit z. B. m = 13, 15, 17, 19 und n = 1, 2, 3 wegen deren Schwer- oder Unlöslichkeit in nichtfluorierten Lösungsmitteln im allgemeinen ungeeignet. Es bestand somit die Aufgabe, eine Methode zur Umwandlung sehr langkettiger Fluoralkohole in grenzflächenaktive Produkte zu finden.

Überraschenderweise wurde gefunden, daß die allgemein reaktionsträgen, sehr langkettigen Fluoralkohole des Typs CF₃(CF₂)ₘ(CH₂)ₙOH mit m = 13, 15, 17, 19 und n = 1, 2, 3 die als technische Zwangsanfall-Produkte in großen Mengen entstehen, durch Umsetzung mit reaktiven Substraten entweder direkt in grenzflächenaktive Stoffe oder aber zuerst in reaktive Zwischenprodukte (Vorstufen grenzflächenaktiver Stoffe) überführt und dann in einfacher Weise in grenzflächenaktive Substanzen umgewandelt werden können. Es liegt dabei im Rahmen der Erfindung, daß diese Reaktionen bevorzugt bei Abwesenheit von Lösungsmitteln durch direkte Einwirkung der Umsetzungspartner auf die festen oder flüssigen fluorhaltigen Ausgangsmaterialien erfolgen und überraschenderweise fast immer quantitative Ausbeuten ergeben.

Gegenstand der Erfindung sind langkettige fluoralkylmodifizierte Kohlenwasserstoffderivate der Formel

R_{F}-(CH₂)ₙ-A-D

in der
- n: 1, 2 oder 3,
- R_{F}: geradkettige Perfluoralkylreste mit 6 bis 20 C-Atomen,
- A: eine Einfachbindung oder eine der Gruppen 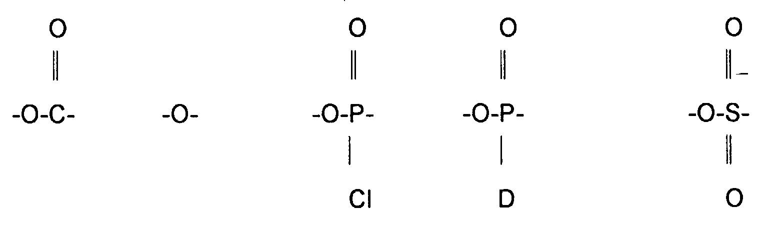
- D: (D₁): geradkettige, verzweigte oder cyclische, gesättigte oder ungesättigte Kohlenwasserstoffreste mit 2 bis 100 C-Atomen, oder
(D₂): Molekülgerüste des Typs -Y(R¹)(R²)(R³) mit
Y = gesättigte oder ungesättigte, verknüpfte oder mehrgliedrig annelierte cyclische Kohlenwasserstoffe mit 4 bis 40 C-Atomen, und
R¹, R², R³ = gleiche oder verschiedene sauerstoff-, schwefel- oder stickstoffhaltige, hydroxylierte oder nichthydroxylierte Kohlenwasserstoffreste mit 2 bis 50 C-Atomen, oder
(D₃): geradkettige, verzweigte oder cyclische, gesättigte oder ungesättigte mono- oder polyhydroxylierte und/oder durch Alkylenoxideinheiten modifizierte, ggf. stickstoffhaltige Kohlenwasserstoffreste mit 2 bis 100 C-Atomen
bedeuten.
Gegebenenfalls bedeuten:
- (D₁): durch Stickstoff, Sauerstoff, Schwefel oder Halogen substituierte, geradkettige, verzweigte oder cyclische, gesättigte oder ungesättigte Kohlenwasserstoffreste mit 2 bis 100 C-Atomen,
- Y: verbrückte oder über ein Heteroatom oder eine C₁-Einheit, die durch Stickstoff, Sauerstoff, Halogen oder halogenierte Alkylreste substituiert sein kann, gesättigte oder ungesättigte, verknüpfte oder mehrgliedrig annelierte cyclische Kohlenwasserstoffe mit 4 bis 40 C-Atomen, und
- R¹, R² und R³: gleiche oder verschiedene, Metallionen, Ammoniumionen oder Wasserstoffionen enthaltende, in 1.2- oder 1.3-Stellung am Ring kovalente oder ionogene Bindungen aufweisende Substituenten oder über zwei weitere, teilweise funktionalisierte Kohlenstoffe mit dem Ring verknüpfte Kohlenwasserstoffreste mit 2 bis 50 C-Atomen.

Die genannten Strukturelemente haben vorzugsweise folgende Bedeutung:
- R_{F}: Perfluoralkyl mit Kettenlängen von C₈ bis C₂₀, besonders C₁₀ bis C₂₀, speziell C₁₂ bis C₁₈, ganz speziell C₁₄ bis C₁₆
- D₁: -O(CH₂)ₙR_{F} mit n und R_{F} wie oben erläutert, -CF₃, -CH₂Cl, -CH₂Br, -CH₂CH₂CH₂Cl, -CH₂CH₂CH₂CH₂Br, -NHCH₂CH₂Cl, -NHCH₂CH₂CH₂Cl, -NCO, -NHC₆H₃(CH₃)NCO, -NH(CH₂)ₖNCO mit k = 1 bis 10, -CHCH₂O, -CH₂CH₂OH, -CH₂CH(OH)CH₃
- Y:

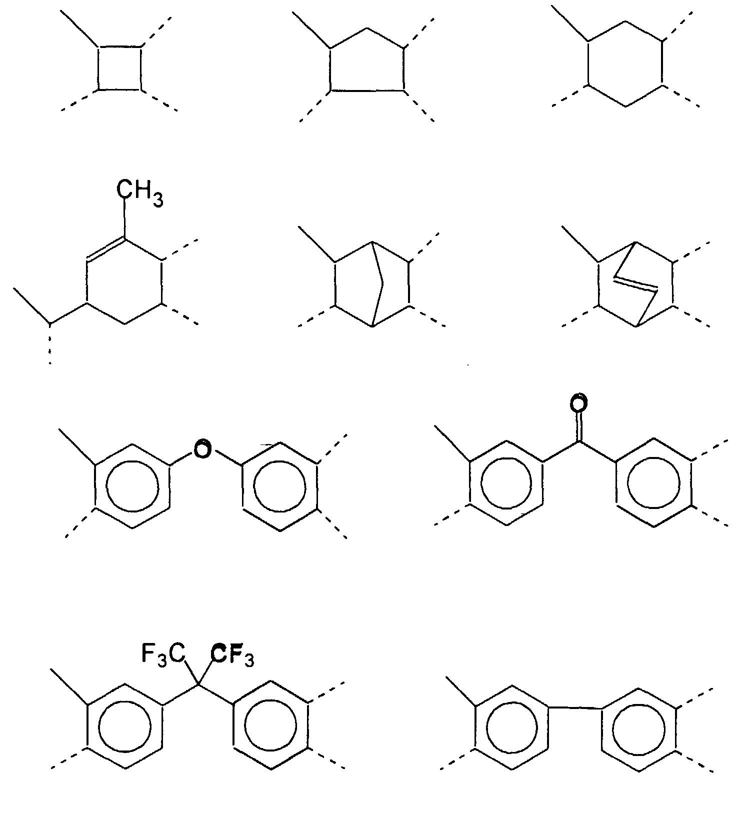

In den Strukturbildern symbolisiert die durchgezogene Linie am Ring (Ringsubstituenten) die Bindung des Strukturparameters Y zum Strukturteil R_{F}(CH₂)ₙ-A. Gestrichelte Linien beschreiben Bindungen von Y zu den Strukturparametern R¹, R², R³.
- R¹,R²,R³: -CO₂H, -SO₃H, -OH, -COO(CH₂)ₙR_{F} mit R_{F} und n wie oben erläutert, -C(=O)NHC(CH₂OH)₃, -C(=O)N(CH₂CH₂OH)₂, -COO⁻M⁺, -SO₃ ⁻M⁺ mit M⁺ = H⁺, Na⁺, K⁺,¹/₂ Mg²⁺, ¹/₂ Ca²⁺, NR₄⁺, NHR₃⁺, NH₂R₂⁺, NH₃R⁺ mit R = H, CH₃, C₂H₅, C₃H₇, C₄H₉, -CH₂CH₂OH, -(CHOH)ᵤCH₂OH, -CH₂(CHOH)ᵤCH₂OH mit u = 3 bis 5, oder einen Rest der Formeln
[HOCH₂CH₂NH₂CH₂CH₂NHC(=O)CH(OH)(HO)CHCH(OH)-CH(OH)CH₂OH]⁺
[H₂N[CH₂CH₂NHC(=O)CH(OH)(HO)CHCH(OH)CH(OH)CH₂OH]₂]⁺
[H₂N-C₄H₈-
NHCH₂CH₂NHC(=O)CH(OH)(HO)CHCH(OH)CH(OH)CH₂OH]⁺
- D₃: -CH(OH)CH₂OH, -[CH(OH)CH₂O]₂CH₂CH(OH)CH₂OH,
-[CH(OH)CH₂O]₃CH₂CH(OH)CH₂OH, CH(OH)(HO)CH(HO)CHCH₂OH,
-CH(OH)(HO)CH(HO)CHCH₂OH,
-CH(OH)(HO)CHCH(OH)CH(OH)CH₂OH,
-(HO)CH(HO)CHCH(OH)CH(OH)CH₂OH,
-CH(OH)(HO)CH(HO)CHCH(OH)CH₂OH,
-N(CH₂CH₂OH)CH(OH)(HO)CHCH(OH)CH(OH)CH₂OH,
-N(CH₃)CH(OH)(HO)CHCH(OH)CH(OH)CH₂OH,
-CH₂N(CH₂CH₂OH)CH(OH)(HO)CHCH(OH)CH(OH)CH₂OH,
(-NH)CH(CH₃)CH₂(OCH₂CH₂)ᵣOCH₂CH(NH₂)CH₃, mit r = 5 bis 2000, vorzugsweise 50 bis 500.

Als Ausgangsmaterialien CF₃(CF₂)ₘ(CH₂)ₙOH für das Strukturelement R_{F}-(CH₂)ₙ⁻werden Reinsubstanzen oder technische Gemische mit m = 5, 7, 9, 11, 13, 15, 17, 19 und n = 1, 2, 3 verwendet. Vorzugsweise kommen Alkohole mit fluorierten Alkylketten der Länge C₈ bis C₂₀, besonders mit Kettenlängen C₁₀ bis C₂₀, speziell mit Kettenlängen C₁₂ bis C₁₈, ganz speziell mit Kettenlängen C₁₄ bis C₁₆ zum Einsatz.

Die Einführung der Strukturelemente A - D erfolgt durch Alkylierung oder Acylierung der Ausgangsmaterialien, wobei das Strukturelement D entweder durch die Struktur des Substrats für die Einführung von A vorgegeben ist oder durch Derivatisierung des bei der Einführung von A erzeugten Produktes resultiert.

Für die Alkylierung oder Acylierung können bevorzugt Dihalogenalkane, Halogenhydrine, di- und höherfunktionelle Epoxide, Phosphoroxy- oder Sulfurylhalogenide, difunktionelle Carbonsäuren, Säurehalogenide, Anhydride oder Isocyanate eingesetzt werden.

Das Strukturelement D = D₁ entsteht, wenn die Alkylierung oder Acylierung mit halogenierten oder nichthalogenierten Epoxiden, halogenierten Säurederivaten, halogenierten Isocyanaten oder Diisocyanaten durchgeführt wird. Exemplarisch seien 1,2-Dibromethan, Epichlorhydrin, Bromacetylbromid, p-Toluolsulfonsäurechlorid Chloressigsäure, Chloressigsäureanhydrid, das gemischte Anhydrid aus Chlor- und Trifluoressigsäure nach Stacey et al., Nature 164 (1949) 705, 2-Chlorethylisocyanat in Ulsperger und Jacob, Fette, Seifen, Anstrichm. 64 (1962) 1093 - 8, 2,4-Toluylendiisocyanat, Chlorcarbonylisocyanat sowie die Umsetzung mit Phosphorylchlorid nach Milius, Greiner, Riess, Carbohydr. Res. 129 (1992) 323 - 36 genannt.

Die Einführung des Strukturelements D = D₂ erfolgt durch Acylierung mit Anhydriden von gesättigten oder ungesättigten Tetra- oder höheren Carbonsäuren, wobei D₂ aus dem das Strukturgerüst (Mittelteil des Dianhydrids) charakterisierenden Strukturparameter Y und den die Gerüstfunktionalitäten beschreibenden Strukturparametern R¹,R²,R³ zusammengesetzt ist. Der Strukturparameter Y wird beispielsweise durch Pyromellitsäuredianhydrid, 5-(2,5-Dioxo-tetrahydrofuryl)-3-methyl-3-cyclohexen-1,2-dicarbonsäureanhydrid, Cyclobutan-1,2,3,4-tetracarbonsäuredianhydrid, Cyclopentan-2,3,4,5-tetracarbonsäuredianhydrid, Cyclohexan-2,3,5,6-tetracarbonsäuredianhydrid, Bicyclo[2.2.2]oct-7-en-2,3,5,6-tetracarbonsäuredianhydrid, Bicyclo[2.1.2]heptan-2,3,5,6-tetracarbonsäuredianhydrid, 3,3',4,4'-Benzophenontetracarbonsäuredianhydrid, 2,2-Bis-(3-phthalsäureanhydrid)hexafluorpropan, 3,3',4,4'-Diphenyloxidtetracarbonsäuredianhydrid oder 3,3',4,4'-Diphenyltetracarbonsäuredianhydrid realisiert. Die Strukturparameter R¹,R²,R³ resultieren durch Freisetzung und Neutralisation der Carbonsäurefunktionen mit stickstoffhaltigen Hydroxylverbindungen oder niedermolekularen, nichthydroxylierten Alkylaminen oder durch Hydrogensulfitaddition an ungesättigte Anhydride. Exemplarisch sei die Reaktion der mit Dianhydriden gebildeten Acylierungsprodukte mit wäßriger Natron- oder Kalilauge oder wäßrigen Lösungen von Ammoniumhydroxid, Tris-(hydroxymethyl)aminomethan oder Diethanolamin genannt.

Die Einführung des Strukturelements D = D₃ erfolgt durch Alkylierung oder Acylierung der fluorhaltigen Ausgangsmaterialien mit Reagenzien, die hydrophilierende Gruppen tragen oder durch Umsetzung mit dem Edukt erzeugen, wie z. B. Glycidol, Glycerol und D-Gluconsäure und/oder durch Derivatisierung der bei der Einführung von A erhaltenen Alkylierungs- und Acylierungsprodukte mit dem Strukturelement D₁ mit durch Reduktion von Zuckern zugänglichen oder anderen, von Zuckern abgeleiteten stickstoffhaltigen polyhydroxylierten Substraten wie beispielsweise Arabit, Sorbit, Mannit, Dulcit, D-Glucamin, N-Methyl- und N-(2-Hydroxyethyl)-D-glucamin sowie O,O'-Bis-(2-aminopropyl)polyethylen- und -polypropylenglycole.

Erfindungsgemäß sind beispielsweise folgende Ausführungsformen zur Erzeugung grenzflächenaktiver Substanzen aus den langkettig fluoralkylmodifizierten Kohlenwasserstoffderivaten:
1) Umsetzung der fluorhaltigen Ausgangsmaterialien mit geeigneten Alkylierungs- oder Acylierungsmitteln zu Fluoralkylethern oder -estern (Vorstufen), die im nichtfluorierten Molekülteil eine weitere reaktive Funktionalität wie beispielsweise Halogen, Hydroxyl oder Isocyanat enthalten, über welche in einem weiteren Reaktionsschritt die Verknüpfung mit einer hydrophilierenden Komponente erfolgt. Als vorteilhafte Alkylierungsmittel sind jeweils doppelt endständig monohalogenierte Alkane wie z. B. 1,2-Dibromethan, 1,3-Dichlorpropan und 1,4-Dibrombutan und Epoxide wie beispielsweise Epichlorhydrin, Epibromhydrin, Ethylenoxid, Propylenoxid, Hexenoxid, Octenoxid und Cyclohexenoxid anzusehen. Entsprechende Acylierungsmittel umfassen halogenierte Säuren, Säurehalogenide, Säureanhydride und Isocyanate, Diisocyanate sowie Phosphoroxy- und Sulfurylhalogenide. Als Beispiele seien Chloressigsäure, 3-Chlorpropionylchlorid, Chloressigsäureanhydrid, 3-Chlorpropylisocyanat, 2,4-Toluylendiisocyanat und Hexamethylendiisocyanat genannt. Als hydrophilierende Systeme bieten sich Mono-, Di- oder Trisaccharide wie z. B. Glucose, Maltose und Raffinose, Aminosaccharide wie z. B. Glucosamin, N-Acetyl-D-glucosamin, D-Glucamin, N-Alkyl-D-glucamine und N-Glucoside, saccharidmodifizierte Aminoamide gemäß DE-OS-4 318 536 Beispiel 11a und Beispiel 26a, aminomodifizierte Ethylen- und Propylenglycole verschiedener Kettenlängen oder allgemein Amino- und Amidoverbindungen mit zahlreichen polaren funktionellen Gruppen wie z. B. N-(2-Acetamino)iminodiessigsäure und Panthenol an. In Abhängigkeit von der Struktur des nichtfluorierten Molekülteils der Vorstufe läßt sich über die Anpassung der hydrophilierenden Komponente eine Verstärkung der Wasser- oder Öllöslichkeit des grenzflächenaktiven Stoffs einstellen.
2) Acylierung der fluorhaltigen Edukte mit Dianhydriden und anschließende Amidierung und Neutralisation nichtveresterter Carbonsäurefunktionen mit Ammoniak und ggf. hydroxylgruppenhaltigen Alkylammoniumverbindungen. Als Acylierungsmittel sind neben niedermolekularen Substraten wie z. B. Pyromellitsäuredianhydrid auch höhergliedrige Systeme wie beispielsweise Naphthalin-1,4,5,8-tetracarbonsäuredianhydrid und Perylen-3,4,9,10-tetracarbonsäuredianhydrid vorteilhaft. Als hydrophilierende Komponenten dienen neben Di- und Triethanolamin Tris-(hydroxymethyl)-aminomethan und dessen Derivate wie vorzugsweise N-Tris-(hydroxymethyl)-methyl-3-amino-2-hydroxy-propansulfonsäure, N-Tris-(hydroxymethyl)-methyl-2-aminoethansulfonsäure, N-Tris-(hydroxymethyl)-methyl-3-aminopropansulfonsäure und N-Tris-(hydroxymethyl)-methylglycin sowie Aminosaccharide und saccharidmodifizierte Aminoamide gemäß DE-OS-4 318 536 Beispiel 11a und Beispiel 26a.
3) Direkte Alkylierung und Acylierung der partiell fluorierten Ausgangsmaterialien zu grenzflächenaktiven Stoffen. Die Umsetzung mit Reaktionspartnern, die bereits eine hinreichende Zahl hydrophilierend wirkender polarer Gruppen enthalten oder solche in mehrfacher Reaktion mit dem Edukt erzeugen, ergibt in einstufiger Synthese Fluortenside und Ausrüstungsmittel. Als bevorzugte Alkylierungsmittel dienen Glycerol, Glycidol und dessen Derivate wie beispielsweise Ethylenglycoldiglycidylether, tert.-Butylglycidylether, Methacrylsäureglycidylester, Isopropylglycidether, Allylglycidether und Phenylglycidether. Als vorteilhafte Acylierungsmittel gelten Zuckercarbonsäuren und deren Lactone wie z. B. D-Gluconsäure, Lactobionsäure, Pectinsäure, Mucinsäure, Gluconsäure-δ-lacton, Heptagluconsäure-γ-lacton und Glucopyranosylarabonsäurelacton, Tri- und Tetracarbonsäuren von Diaminen wie z. B. N-(2-Hydroxyethyl)ethylendiamin-N,N',N'-triessigsäure, Ethylen- und Propylendiamin-tetraessigsäure und andere Carbonsäuren oder Anhydride, die hydrophile Gruppen tragen wie beispielsweise 3,6,9-Trioxaundecandisäure N,N-Bis-(2-hydroxyethyl)glycin, N-Tris-(hydroxymethyl)-methylglycin, Aconitsäureanhydrid oder Di-O-acetyl-L-weinsäureanhydrid. Die Umsetzungen der fluorierten Ausgangsmaterialien mit Alkylierungs- oder Acylierungsmitteln erfolgen unter Rühren bei Abwesenheit eines Lösungsmittels bei Normaldruck oder erhöhtem Druck und Temperaturen von 20 °C bis 200 °C, bevorzugt zwischen 50 °C und 150 °C. Alkylierungen und Acylierungen werden in Gegenwart von basischen oder sauren Katalysatoren durchgeführt. Als basische Katalysatoren sind Alkalien, niedermolekulare Alkylamine und Stickstoffheterocyclen bevorzugt. Besonders vorteilhaft sind Natrium- und Kaliumhydroxid, Triethylamin oder Imidazol. Als saure Katalysatoren eignen sich starke Säuren wie beispielsweise Schwefelsäure p-Toluolsulfonsäure, Methansulfonsäure oder Trifluoressigsäure.

Bei der zweistufigen Synthese grenzflächenaktiver Stoffe ist eine Isolierung der gebildeten Zwischenprodukte in der Regel nicht erforderlich.

In Ausnahmefällen, wie beispielsweise bei der Erzeugung grenzflächenaktiver Stoffe aus phosphorylierten Fluoralkoholen ist die Verwendung eines aprotischen Lösungsmittels günstig. Besonders vorteilhaft sind Diethylether und Tetrahydrofuran.

Die Neutralisation der aus Fluoralkoholen und speziellen Dianhydriden erhaltenen Produkte mit stickstoffhaltigen Hydroxylverbindungen verläuft in Wasser oder niederen Alkoholen wie z. B. Methanol und Isopropanol beschleunigt.

Die erfindungsgemäßen Verbindungen werden als grenzflächenaktive Stoffe und Ausgangsstoffe für die Herstellung von Verbindungen für die öl- und schmutzabweisende Ausrüstung von Textilien, Leder, Holz oder Papier verwendet.

Weitere Einzelheiten der Reaktionsdurchführung sind den Beispielen zu entnehmen:

### Beispiel 1

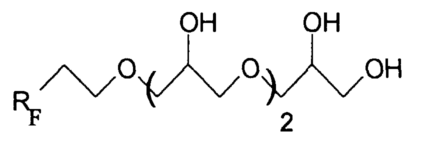

10.0 g Fluoralkohol mit R_{F} = C₁₄-C₂₀ und 3.9 g Glycidol werden in Gegenwart von 1.0 g Kaliumhydroxid im Stahlautoklav 9.5 h auf 140-150 °C erhitzt. Die resultierende klebrige braune Masse wird in heißem Wasser aufgenommen und filtriert. Das stark schäumende dunkelbraune undurchsichtige Filtrat ergibt beim Einengen 7.2 g eines hellbraunen klebrigen Feststoffs.

Die Oberflächenspannung einer wäßrigen Lösung mit 1 g/l beträgt 25 mN/m bei 20 °C.
IR (cm⁻¹) 3400 (br.), 2880, 1620, 1200, 1150, 550, 520

### Beispiel 2a

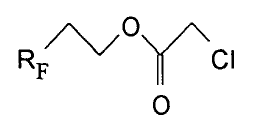

29.7 g Fluoralkohol mit R_{F} = C₁₀-C₂₀ und 13.6 g Chloressigsäureanhydrid werden 2 h auf 80 - 90 °C erhitzt. Beim Eingeben der heißen Reaktionslösung in gesättigte wäßrige Natriumhydrogencarbonatlösung scheiden sich 29.5 g eines gelben, körnerartigen Feststoffs ab.
¹H-NMR (ppm) 2.56-2.71 (tt, J = 6.0 Hz, 2H, R_{F}CH₂CH₂O), 4.19 (s, 2H, O₂CCH₂Cl), 4.47 (t, 2H, J = 6.0 Hz, R_{F}CH₂CH₂O)

### Beispiel 2b

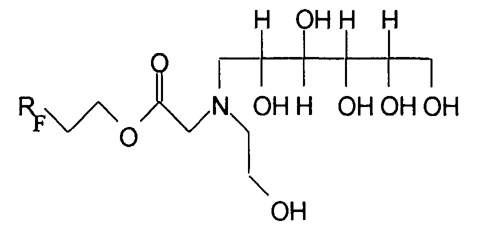

10.0 g Chloressigsäureperfluoralkylethylester und 3.5 g N-(2-Hydroxyethyl)-D-glucamin werden in 200 ml Tetrahydrofuran insgesamt 6 h auf Rückflußtemperatur erhitzt. Beim Einengen der Reaktionslösung verbleiben 12.5 g eines schwach gelben, schmierigen Feststoffs.

Die Oberflächenspannung einer wäßrigen Lösung mit 1 g/l beträgt 30 mN/m bei 20 °C.
IR (cm⁻¹) 3400 (br.), 2900, 1610, 1400 (br.), 1200, 1150, 1010, 650, 550

### Beispiel 3

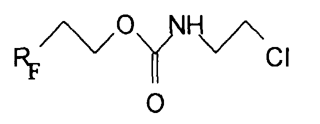

10.0 g Fluoralkohol mit R_{F} = C₁₄-C₂₀ und 2.3 g 2-Chlorethylisocyanat werden 30 h auf 80 °C erhitzt. Nach dem Abkühlen verbleiben 11.1 g einer farblosen festen Masse, die sich zu Pulver zermörsern läßt.
¹H-NMR (ppm) 2.48-2.61 (m, 2H, R_{F}CH₂CH₂O), 3.39 (m, 2H, NHCH₂CH₂Cl), 3.56 (m, 2H, NHCH₂CH₂Cl), 4.47 (t, 2H, J = 6.2 Hz, R_{F}CH₂CH₂O), 6.89 (s, br. 1H, NH)

### Beispiel 4

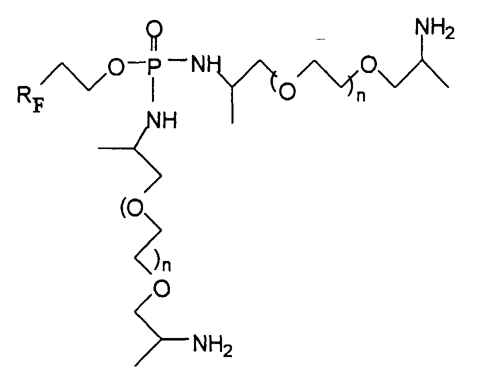

2.0 g Phosphorsäuremonoperfluoralkylethylesterdichlorid dargestellt aus Fluoralkohol mit R_{F} = C₁₄-C₁₆ und Phosphorylchlorid entspechend Milius, Greiner, Riess, Carbohydr. Res. 129 (1992) 323-36 und 11.7 g O,O'-Bis-(2-aminopropyl)-polyethylenglycol 1900 werden in 100 ml Tetrahydrofuran 6 h auf Rückflußtemperatur erhitzt. Beim Einengen der Reaktionslösung verbleiben 12.5 g eines hellbraunen, viskosen Öls, das wasserlöslich ist und schäumt.

Die Oberflächenspannung einer wäßrigen Lösung mit 1 g/l beträgt 31 mN/m bei 20 °C.
IR (cm⁻¹) 3400 (br.), 2880, 1620, 1480, 1350, 1280, 1220, 1100, 950, 830

### Beispiel 5

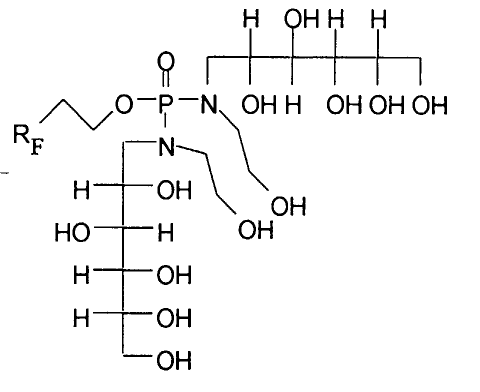

2.0 g Phosphorsäuremonoperfluoralkylethylesterdichlorid (dargestellt aus Fluoralkohol mit R_{F} = C₁₄-C₂₀) und 1.3 g N-(2-Hydroxyethyl)-D-glucamin werden in 150 ml Tetrahydrofuran in Gegenwart von 1.2 g Triethylamin 10 h auf Rückflußtemperatur erhitzt. Nach Filtration und Einengen der Reaktionslösung resultieren 2.7 g eines schwach gelben Feststoffs, der wasserlöslich ist und schäumt.

Die Oberflächenspannung einer wäßrigen Lösung mit 1 g/l beträgt 24 mN/m bei 20 °C.
IR (cm⁻¹) 3300, 2900, 2600, 2480 (br.), 1590, 1450, 1200, 1050 (br.), 850, 800, 650, 550, 520

### Beispiel 6a

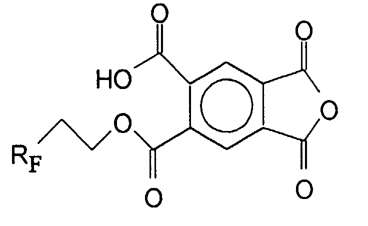

22.8 g Fluoralkohol mit R_{F} = C₁₀-C₂₀ und 8.4 g Pyromellitsäuredianhydrid werden in Gegenwart von 0.3 g Triethylamin 7 h auf 150 °C erhitzt. Die nach dem Abkühlen verbleibende farblose feste Masse läßt sich zu 29.8 g Pulver zermörsern.
¹H-NMR (ppm) 2.73 (m, 2H, R_{F}CH₂CH₂O), 4.61 (t, J = 6.2 Hz, 2H, R_{F}CH₂CH₂O), 8.03 (s, 2H ArH), 10.84 (s, br. 1H, COOH)

### Beispiel 6b

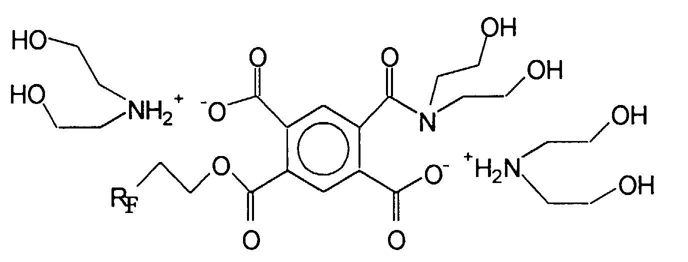

5.0 g Anhydridperfluoralkylethylester und 2.0 g Diethanolamin werden in 120 ml Isopropanol 4 h auf Rückflußtemperatur erhitzt. Nach Filtration von unlöslichen Bestandteilen und Einengen des Filtrats verbleiben 3.7 g einer hellgelben, schmierigen Paste, die wasserlöslich ist und schäumt.

Die Oberflächenspannung einer wäßrigen Lösung mit 5 g/l beträgt 31 mN/m bei 20 °C.
IR (cm⁻¹) 3300 (br.), 2900 (br.), 1710, 1600, 1390 (br.), 1200 (br.), 1050, 950, 800, 700, 650, 550, 520

### Beispiel 7

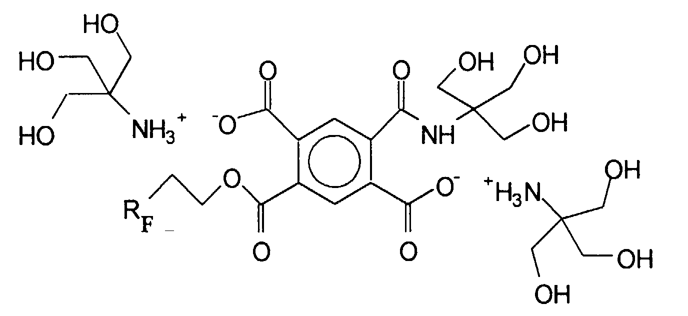

13.7 g Anhydridperfluoralkylethylester, dargestellt aus Fluoralkohol mit R_{F} = C₁₄-C₁₆ und 6.2 g Tris-(hydroxymethyl)-aminomethan, werden in 250 ml Wasser 1 h unter Rückfluß zum Sieden erhitzt. Beim Einengen der milchig-trüben, stark schäumenden wäßrigen Lösung verbleiben 17.5 g eines farblosen, klebrigen Feststoffs.

Die Oberflächenspannung einer wäßrigen Lösung mit 5 g/l beträgt 33 mN/m bei 20 °C.
IR (cm⁻¹) 3400, 1700, 1610, 1580, 1390, 1320, 1200 (br.), 1150, 650

### Beispiel 8

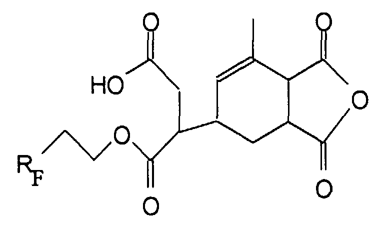

10.0 g Fluoralkohol mit R_{F} = C₁₄-C₂₀ und 5.7 g Epiclon B-4400 werden in Gegenwart von 0.1 g Triethylamin 7 h auf 150 °C erhitzt. Die beim Abkühlen resultierende gelb-braune feste Masse läßt sich zu 14.0 g Pulver zermörsern, das wasserlöslich ist und stark schäumt.

Die Oberflächenspannung einer wäßrigen Lösung mit 1 g/l beträgt 18 mN/m bei 20 °C.
IR (cm⁻¹) 3400, 2980 (br.), 1850, 1760, 1720, 1620, 1200 (br.), 1150, 650

## Patentansprüche

1. Langkettige fluoralkylmodifizierte Kohlenwasserstoffderivate der Formel
R_{F}-(CH₂)ₙ-A-D
in der
n 1, 2 oder 3,
R_{F} geradkettige Perfluoralkylreste mit 6 bis 20 C-Atomen,
A eine Einfachbindung oder eine der Gruppen 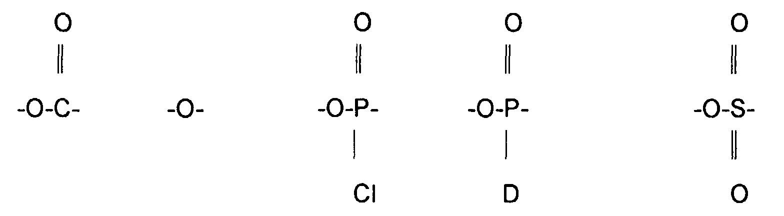
D
(D₁): geradkettige, verzweigte oder cyclische, gesättigte oder ungesättigte Kohlenwasserstoffreste mit 2 bis 100 C-Atomen, oder
(D₂): Molekülgerüste des Typs -Y(R¹)(R²)(R³) mit
Y = gesättigte oder ungesättigte, verknüpfte oder mehrgliedrig annelierte cyclische Kohlenwasserstoffe mit 4 bis 40 C-Atomen, und
R¹, R², R³ = gleiche oder verschiedene sauerstoff-, schwefel- oder stickstoffhaltige, hydroxylierte oder nichthydroxylierte Kohlenwasserstoffreste mit 2 bis 50 C-Atomen, oder
(D₃): geradkettige, verzweigte oder cyclische, gesättigte oder ungesättigte mono- oder polyhydroxylierte und/oder durch Alkylenoxideinheiten modifizierte, ggf. stickstoffhaltige Kohlenwasserstoffreste mit 2 bis 100 C-Atomen
bedeuten.

2. Verbindungen gemäß Anspruch 1, dadurch gekennzeichnet, daß (D₁) durch Stickstoff, Sauerstoff, Schwefel oder Halogen substituierte, geradkettige, verzweigte oder cyclische, gesättigte oder ungesättigte Kohlenwasserstoffreste mit 2 bis 100 C-Atomen bedeutet.

3. Verbindungen gemäß den Ansprüchen 1 und/oder 2, dadurch gekennzeichnet, daß Y verbrückte oder über ein Heteroatom oder eine C₁-Einheit, die durch Stickstoff, Sauerstoff, Halogen oder halogenierte Alkylreste substituiert sein kann, gesättigte oder ungesättigte, verknüpfte oder mehrgliedrig annelierte cyclische Kohlenwasserstoffe mit 4 bis 40 C-Atomen bedeutet.

4. Verbindungen gemäß einem oder mehreren der Ansprüche 1 bis 3, dadurch gekennzeichnet, daß R¹, R² und R³ gleiche oder verschiedene, Metallionen, Ammoniumionen oder Wasserstoffionen enthaltende, in 1.2- oder 1.3-Stellung am Ring kovalente oder ionogene Bindungen aufweisende Substituenten oder über zwei weitere, teilweise funktionalisierte Kohlenstoffe mit dem Ring verknüpfte Kohlenwasserstoffreste mit 2 bis 50 C-Atomen bedeuten.

5. Verbindungen gemäß einem oder mehreren der Ansprüche 1 bis 4, dadurch gekennzeichnet, daß unabhängig voneinander
R_{F} Perfluoralkyl mit Kettenlängen von C₈ bis C₂₀, besonders C₁₀ bis C₂₀, speziell C₁₂ bis C₁₈, ganz speziell C₁₄ bis C₁₆ ;
D₁ -O(CH₂)ₙR_{F}, -CF₃, -CH₂Cl, -CH₂Br,
-CH₂CH₂CH₂Cl, -CH₂CH₂CH₂CH₂Br, -NHCH₂CH₂Cl,
-NHCH₂CH₂CH₂Cl, -NCO, -NHC₆H₃(CH₃)NCO,
-NH(CH₂)ₖNCO mit k = 1 bis 10, -CHCH₂O, -CH₂CH₂OH,
-CH₂CH(OH)CH₃;
Y 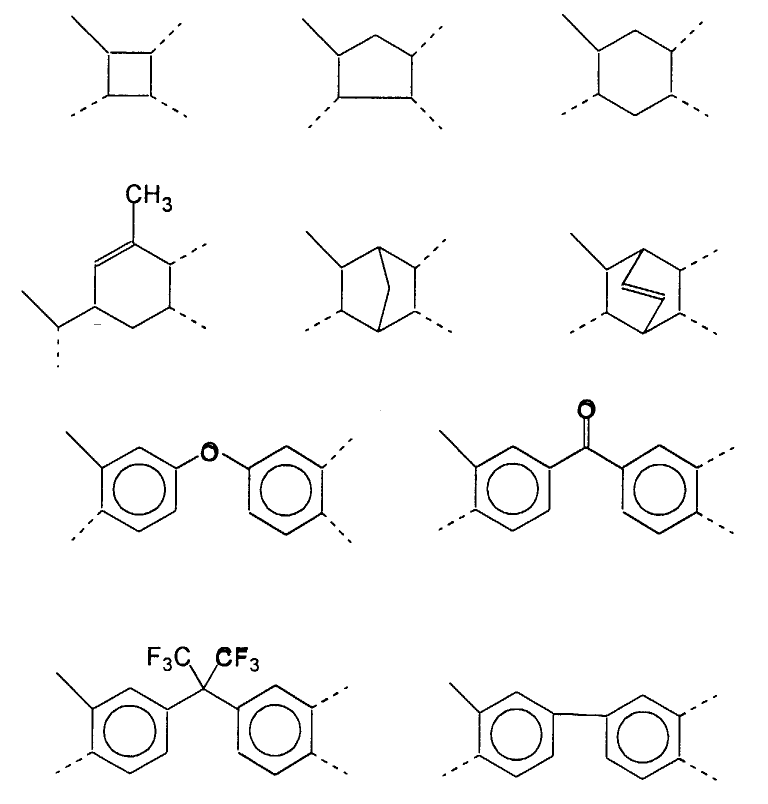 worin die durchgezogenen Linien am Ring (Ringsubstituenten) die Bindung des Strukturparameters Y zum Strukturteil R_{F}(CH₂)ₙ-A und gestrichelte Linien Bindungen von Y zu den Strukturparametern R¹, R², R³ darstellen;
R¹,R²,R³ -CO₂H, -SO₃H, -OH, -COO(CH₂)ₙR_{F}, -C(=O)NHC(CH₂OH)₃, - C(=O)N(CH₂CH₂OH)₂, -COO⁻M⁺, -SO₃ ⁻M⁺ mit M⁺ = H⁺, Na⁺, K⁺,¹/₂ Mg²⁺, ¹/₂ Ca²⁺, NR₄⁺, NHR₃⁺, NH₂R₂⁺, NH₃R⁺ mit R = H, CH₃, C₂H₅, C₃H₇, C₄H₉, -CH₂CH₂OH, -(CHOH)ᵤCH₂OH, CH₂(CHOH)ᵤCH₂OH mit u = 3 bis 5,
oder einen Rest der Formeln
[HOCH₂CH₂NH₂CH₂CH₂NHC(=O)CH(OH)(HO)CHCH(OH)-CH(OH)CH₂OH]⁺
[H₂N[CH₂CH₂NHC(=O)CH(OH)(HO)CHCH(OH)CH(OH)CH₂OH]₂]⁺
[H₂N-C₄H₈-
NHCH₂CH₂NHC(=O)CH(OH)(HO)CHCH(OH)CH(OH)CH₂OH]⁺
D₃ -CH(OH)CH₂OH, -[CH(OH)CH₂O]₂CH₂CH(OH)CH₂OH,
-[CH(OH)CH₂O]₃CH₂CH(OH)CH₂OH, CH(OH)(HO)CH(HO)CHCH₂OH,
-CH(OH)(HO)CH(HO)CHCH₂OH,
-CH(OH)(HO)CHCH(OH)CH(OH)CH₂OH,
-(HO)CH(HO)CHCH(OH)CH(OH)CH₂OH,
-CH(OH)(HO)CH(HO)CHCH(OH)CH₂OH,
-N(CH₂CH₂OH)CH(OH)(HO)CHCH(OH)CH(OH)CH₂OH,
-N(CH₃)CH(OH)(HO)CHCH(OH)CH(OH)CH₂OH,
-CH₂N(CH₂CH₂OH)CH(OH)(HO)CHCH(OH)CH(OH)CH₂OH,
(-NH)CH(CH₃)CH₂(OCH₂CH₂)ᵣOCH₂CH(NH₂)CH₃, mit r = 5 bis 2000, vorzugsweise 50 bis 500
bedeuten.

6. Verbindungen gemäß einem oder mehreren der Ansprüche 1 bis 5, dadurch gekennzeichnet, daß sich die geradkettigen, verzweigten oder cyclischen gesättigten oder ungesättigten, ggf. durch Stickstoff, Sauerstoff, Schwefel oder Halogen substituierten Kohlenwasserstoffe im Strukturelement D₁ von Verbindungen aus den Gruppen der Dihalogenalkane, Halogenhydrine, Di- oder höheren Epoxide, Phosphoroxy- und Sulfurylhalogenide, halogenierten Carbonsäuren, halogenierten Säurehalogenide, halogenierten Anhydride, halogenierten Isocyanate und Diisocyanate ableiten.

7. Verbindungen gemäß einem oder mehreren der Ansprüche 1 bis 6, dadurch gekennzeichnet, daß sich die geradkettigen, verzweigten oder cyclischen gesättigten oder ungesättigten, ggf. durch Stickstoff, Sauerstoff, Schwefel oder Halogen substituierten Kohlenwasserstoffe in Strukturelement D₁ von den Verbindungen 1,2-Dibromethan, 1,3-Dichlorpropan, 1,4-Dibrombutan, 3-Chlor-1,2-propandiol, Epichlorhydrin, Epibromhydrin, Ethylenoxid, Propylenoxid, Hexenoxid, Octenoxid, Cyclohexenoxid, Phosphorylchlorid, Sulfurylchlorid, p-Toluolsulfonsäurechlorid, Chloressigsäure, 3-Chlorpropionsäure, Bromacetylbromid, 3-Chlorpropionylchlorid, Chloressigsäureanhydrid, Chloressigsäure-Trifluoressigsäureanhydrid, 2-Chlorethylisocyanat, 3-Chlorethylisocyanat, Chlorcarbonylisocyanat, 2,4-Toluylendiisocyanat und Hexamethylendiisocyanat ableiten.

8. Verbindungen gemäß einem oder mehreren der Ansprüche 1 bis 7, dadurch gekennzeichnet, daß sich die gesättigten oder ungesättigten, ggf. verbrückten oder über ein Heteroatom oder eine C₁-Einheit, die durch Stickstoff, Sauerstoff, Halogen oder halogenierte Alkylreste substituiert sein kann, verknüpften oder mehrgliedrig annelierten cyclischen Kohlenwasserstoffe in Strukturparameter Y von Strukturelement D₂ von Verbindungen aus den Gruppen der Anhydride gesättigter oder ungesättigter Tetra- oder höherer Carbonsäuren und die sauerstoff-, schwefel- oder stickstoffhaltigen, hydroxylierte oder nichthydroxylierte Kohlenwasserstoffreste mit 2 bis 50 C-Atomen oder Metall-, Ammonium- oder Wasserstoffionen enthaltenden, kovalente oder ionogene Bindungen aufweisenden, in 1.2- oder 1.3-Stellung am Ring oder über zwei weitere, ggf. funktionalisierte Kohlenstoffe, mit dem Ring verknüpften Substituenten in Strukturparameter R¹,R²,R³ von Strukturelement D₂ von Verbindungen aus den Gruppen der hydroxysubstituierten Alkylamine, niedermolekularen, nichthydroxylierten Alkylamine, Aminosaccharide und saccharidmodifizierten Aminoamide ableiten.

9. Verbindungen gemäß einem oder mehreren der Ansprüche 1 bis 8, dadurch gekennzeichnet, daß sich die gesättigten oder ungesättigten, ggf. verbrückten oder über ein Heteroatom oder eine C₁-Einheit, die durch Stickstoff, Sauerstoff, Halogen oder halogenierte Alkylreste substituiert sein kann, verknüpften oder mehrgliedrig annelierten cyclischen Kohlenwasserstoffe in Strukturparameter Y von Strukturelement D₂ von den Verbindungen Pyromellitsäuredianhydrid, 5-(2,5-Dioxotetrahydrofuryl)-3-methyl-3-cyclohexen-1,2-dicarbonsäureanhydrid, Cyclobutan-1,2,3,4-tetracarbonsäuredianhydrid, Cyclopentan-2,3,4,5-tetracarbonsäuredianhydrid, Cyclohexan-2,3,5,6-tetracarbonsäuredianhydrid, Bicyclo[2.2.2]oct-7-en-2,3,5,6-tetracarbonsäuredianhydrid, Bicyclo[2.1.2]heptan-2,3,5,6-tetracarbonsäuredianhydrid, 3,3',4,4'-Benzophenontetracarbonsäuredianhydrid, 2,2-Bis-(3-phthalsäureanhydrid)hexafluorpropan, 3,3',4,4'-Diphenyloxidtetracarbonsäuredianhydrid oder 3,3',4,4'-Diphenyltetracarbonsäuredianhydrid, Naphthalin-1,4,5,8-tetracarbonsäuredianhydrid und Perylen-3,4,9,10-tetracarbonsäuredianhydrid und die sauerstoff-, schwefel- oder stickstoffhaltigen, hydroxylierte oder nichthydroxylierte Kohlenwasserstoffreste mit 2 bis 50 C-Atomen oder Metallionen enthaltenden, kovalente oder ionogene Bindungen aufweisenden, in 1.2- oder 1.3-Stellung am Ring oder über zwei weitere, ggf. funktionalisierte Kohlenstoffe, mit dem Ring verknüpften Substituenten in Strukturparameter R¹,R²,R³ von Strukturelement D₂ von den Verbindungen Diethanolamin, Triethanolamin, Tris-(hydroxymethyl)-aminomethan, N-Tris-(hydroxymethyl)-methyl-3-amino-2-hydroxy-propansulfonsäure, N-Tris-(hydroxymethyl)-methyl-2-aminoethansulfonsäure, N-Tris-(hydroxymethyl)-methyl-3-amino-propansulfonsäure und N-Tris-(hydroxymethyl)-methylglycin, Methylamine, Ethylamine, Propylamine und Butylamine, Glucosamin N-Acetyl-D-glucosamin, D-Glucamin, N-Methyl-D-glucamin und N-(2-Hydroxyethyl)-D-glucamin ableiten.

10. Verbindungen gemäß einem oder mehreren der Ansprüche 1 bis 9, dadurch gekennzeichnet, daß sich die geradkettigen, verzweigten oder cyclischen, gesättigten oder ungesättigten mono- oder polyhydroxylierten und/oder durch Alkylenoxideinheiten modifizierten, ggf. stickstoffhaltigen Kohlenwasserstoffe in Strukturelement D₃ von Verbindungen aus den Gruppen der hydroxysubstituierten Epoxide und deren Abkömmlinge, Zuckercarbonsäuren und deren Lactone, Tri- und Tetracarbonsäuren von Diaminen, hydrophilierende Substituenten tragenden Carbonsäuren, hydrophilierende Substituenten tragenden Anhydride, Pentosen und Hexosen, Mono-, Di- und Trisaccharide, Aminosaccharide, aminomodifizierten Glycole und hydrophilierende Substituenten tragenden Amino- und Amidoverbindungen ableiten.

11. Verbindungen gemäß einem oder mehreren der Ansprüche 1 bis 10, dadurch gekennzeichnet, daß sich die geradkettigen, verzweigten oder cyclischen, gesättigten oder ungesättigten mono- oder polyhydroxylierten und/oder durch Alkylenoxideinheiten modifizierten, ggf. stickstoffhaltigen Kohlenwasserstoffe in Strukturelement D₃ von den Verbindungen Glycidol, Ethylenglycoldiglycidylether, tert.-Butylglycidylether, Methacrylsäureglycidylester, Isopropylglycidether, Allylglycidether, Phenylglycidether, Glycerol, D-Gluconsäure, Gluconsäure-δ-lacton, Heptagluconsäure-γ-lacton und Glucopyranosylarabonsäurelacton, Lactobionsäure, Pectinsäure, Mucinsäure, N-(2-Hydroxyethyl)ethylendiamin-N,N',N'-triessigsäure, Ethylendiamintetraessigsäure, Propylendiamintetraessigsäure, 3,6,9-Trioxaundecandisäure, N,N-Bis-(2-hydroxyethyl)glycin, N-Tris-(hydroxymethyl)-methylglycin, Aconitsäureanhydrid, Di-O-acetyl-L-weinsäureanhydrid, Arabit, Sorbit, Mannit, Dulcit, Glucose, Maltose, Raffinose, Glucosamin, N-Acetyl-D-glucosamin, D-Glucamin, N-Methyl-D-glucamin, N-(2-Hydroxyethyl)-D-glucamin, O,O'-Bis-(2-aminopropyl)polyethylenglycol, O,O'-Bis-(2-aminopropyl)polypropylenglycol, N-(2-Acetamino)iminodiessigsäure und Panthenol ableiten.

12. Verfahren zur Herstellung von Verbindungen nach einem oder mehreren der Ansprüche 1 bis 11, dadurch gekennzeichnet, daß Verbindungen der Zusammensetzung
R_{F}(CH₂)ₙOH
in der R_{F} und n die oben angegebenen Bedeutungen haben unter Rühren in Substanz bei Abwesenheit eines Lösungsmittels bei 20 °C bis 200 °C, bevorzugt zwischen 50 °C und 150 °C unter Normaldruck oder erhöhtem Druck ggf. in Gegenwart eines Katalysators mit reaktiven Substraten zu reaktiven Zwischenstufen (Vorstufen grenzflächenaktiver Stoffe) oder direkt zu grenzflächenaktiven Stoffen umgesetzt werden.

13. Verfahren nach Anspruch 12, dadurch gekennzeichnet, daß als reaktive Substrate Verbindungen aus den Gruppen der Dihalogenalkane, Phosphoroxy- und Sulfurylhalogenide, halogenierten Carbonsäuren, halogenierten Säurehalogenide, halogenierten Anhydride, Anhydride gesättigter oder ungesättigter Tetra- oder höherer Carbonsäuren, halogenierten Isocyanate, Diisocyanate, hydroxysubstituierten Epoxide und deren Abkömmlinge, Zuckercarbonsäure und deren Lactone, Tri- und Tetracarbonsäuren von Diaminen, hydrophilierende Substituenten tragenden Carbonsäuren, hydrophilierende Substituenten tragende Anhydride verwendet werden.

14. Verfahren nach Anspruch 12 und/oder 13, dadurch gekennzeichnet, daß als basischer Katalysator Natrium- oder Kaliumhydroxid, Triethylamin oder Imidazol oder als saurer Katalysator Schwefelsäure, p-Toluolsulfonsäure, Methansulfonsäure oder Trifluoressigsäure verwendet wird.

15. Verfahren nach einem oder mehreren der Ansprüche 12 bis 14, dadurch gekennzeichnet, daß bei der Reaktion von Verbindungen der Formel
R_{F}(CH₂)ₙOH
in der R_{F} und n die oben angegebenen Bedeutungen haben mit reaktiven Substraten aus den Gruppen der Dihalogenalkane, Halogenhydrine, der Di- oder höheren Epoxide, Phosphoroxy- und Sulfurylhalogenide, halogenierten Carbonsäuren, halogenierten Säurehalogenide, halogenierten Anhydride, halogenierten Isocyanate, Diisocyanate, Anhydride gesättigter oder ungesättigter Tetra- oder höherer Carbonsäuren reaktive Zwischenprodukte (Vorstufen grenzflächenaktiver Stoffe) mit Funktionalität im nichtfluorierten Molekülteil entstehen, die durch Umsetzung mit Verbindungen aus den Gruppen der Mono-, Di- und Trisaccharide, Aminosaccharide aminomodifizierten Glycole, hydroxysubstituierten Alkylamine, niedermolekularen, nichthydroxylierten Alkylamine, Aminosaccharide und saccharidmodifizierten Aminoamide in einem zweiten Syntheseschritt in grenzflächenaktive Stoffe umgewandelt werden.

16. Verfahren nach einem oder mehreren der Ansprüche 12 bis 15, dadurch gekennzeichnet, daß bei der Reaktion von Verbindungen der Zusammensetzung
R_{F}(CH₂)ₙOH
in der R_{F} und n die oben angegebenen Bedeutungen haben, mit reaktiven Substraten aus den Gruppen der hydroxysubstituierten Epoxide und deren Abkömmlinge, Zuckercarbonsäure und deren Lactone, Tri- und Tetracarbonsäuren von Diaminen, hydrophilierende Substituenten tragenden Carbonsäuren, hydrophilierende Substituenten tragenden Anhydride, Anhydride gesättigter oder ungesättigter Tetra- oder höherer Carbonsäuren direkt in einem Syntheseschritt grenzflächenaktive Stoffe erzeugt werden.

17. Verwendung von Verbindungen nach einem oder mehreren der Ansprüche 1 bis 11 als grenzflächenaktive Stoffe und Ausgangsstoffe für die Herstellung von Verbindungen für die öl- und schmutzabweisende Ausrüstung von Textilien, Leder, Holz oder Papier.
